(19)

**Europäisches Patentamt**
**European Patent Office**
**Office européen des brevets**

(11) **EP 1 793 350 B1**

(12) **EUROPEAN PATENT SPECIFICATION**

(45) Date of publication and mention
of the grant of the patent:
**30.04.2014 Bulletin 2014/18**

(51) Int Cl.:
**G06T 7/00** (2006.01)

(21) Application number: **06024725.1**

(22) Date of filing: **29.11.2006**

(54) **Ultrasound imaging system and method for forming a 3D ultrasound image of a target object**

Bilderzeugendes Ultraschallsystem und Verfahren zur Erzeugung eines 3D Ultraschallbildes von einem Zielobjekt

Système de formation d'image à ultrasons et procédé pour former une image en 3D à l'aide d'ultrasons d'un objet cible

(84) Designated Contracting States:
**DE FR IT**

(30) Priority: **01.12.2005 KR 20050116319**

(43) Date of publication of application:
**06.06.2007 Bulletin 2007/23**

(73) Proprietor: **MEDISON CO., LTD.**
**Kangwon-do 250-870 (KR)**

(72) Inventors:
• **Kim, Nam Chul**
**Daegu 706-767 (KR)**
• **Kim, Sang Hyun**
**Busan 612-778 (KR)**
• **Kwon, Eui Chul**
**Seoul 135-280 (KR)**
• **Nguyen, Tien Dung**
**Hanoi (VN)**

(74) Representative: **Lorenz, Markus**
**Lorenz & Kollegen**
**Patentanwälte Partnerschaftsgesellschaft**
**Alte Ulmer Strasse 2**
**89522 Heidenheim (DE)**

(56) References cited:
**EP-A- 1 582 150**

• **THOMAS R. NELSON, DOLORES H. PRETORIUS: "Three-Dimensional Ultrasound Imaging" ULTRASOUND IN MED. & BIOL., [Online] vol. 24, no. 9, 1998, pages 1243-1270, XP002423911 USA Retrieved from the Internet: URL:http://3dultrasound.ucsd.edu/Publicati ons_ files/UMB_V24_1243-1270_1998_TRN-1.pdf > [retrieved on 2007-03-08]**
• **TIEN DUNG NGUYEN ET AL: "An Automatic Body ROI Determination for 3D Visualization of a Fetal Ultrasound Volume" 9TH INTERNATIONAL CONFERENCE, KES 2005, MELBOURNE, AUSTRALIA, SEPTEMBER 14-16, 2005, PROCEEDINGS, PART II, 14 September 2005 (2005-09-14), pages 145-153, XP019015490**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present application claims priority from Korean Patent Application 10-2005 -116319 filed on December 1, 2005.

## BACKGROUND

### 1. Field

The present invention generally relates to ultrasound imaging systems, and more particularly to an ultrasound imaging system and method for forming a 3D ultrasound image of a target object.

### 2. Background

An ultrasound diagnostic system has become an important and popular diagnostic tool due to its wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional (2D or 3D) diagnostic images of a target object. The ultrasound diagnostic system generally uses a wide bandwidth transducer to transmit and receive ultrasound signals. The ultrasound diagnostic system forms ultrasound images of the internal structures of the target object by electrically exciting the transducer to generate ultrasound pulses that travel into the target object. The ultrasound pulses produce ultrasound echoes since they are reflected from a discontinuous surface of acoustic impedance of the internal structure, which appears as discontinuities to the propagating ultrasound pulses. The various ultrasound echoes return to the transducer and are converted into electrical signals, which are amplified and processed to produce ultrasound data for an image of the internal structure.

Document "Three-Dimensional Ultrasound Imaging" by Thomas R Nelson and Dolores H Pretorius, Ultrasound in Med. & Biol., Vol. 24, No. 9, pp. 1243 - 1270, Elsevier 1998, describes and ultrasound imaging system and a method for forming an ultrasound image in which data is classified by choosing a threshold for use with a surface-fitting algorithm and choosing color and opacity for use with volumerendering algorithm.

From EP 1 582 150 A1 an apparatus and a method for forming a 3D ultrasound image is known performing binarization for a fetal image and removing noise region based on brightness values in the binarization regions.

"An Automatic Body ROI Determination for 3D Visualization of a Fetal Ultrasound Volume" by Tien Dung Nguyen et al., Proceedings KES 2005, 14 Sept. 2005, Springer, LNAI 3682, pp. 145 - 153, Berlin Heidelberg 2005, describes a method to determine a body region-of interest (ROI) enclosing a fetus m a two-dimensional key frame that removes some irrelevant matters such as the abdominal area in front of a fetus to visualize a fetal ultrasound volume along with the key frame.

Recently, a 3D ultrasound imaging system has been developed to significantly influence obstetrical diagnosis. Such a system allowed improved observations of complex anatomical structures, surface scan analyses of defects, volumetric measurements of organs and 3D examinations of skeletons. Further, the 3D ultrasound imaging system can generate transparent views, which depict the sculpture-like reconstruction of fetal surface structures and transparent images of fetal inner anatomy.

A 3D ultrasound image of a fetus contains a significant amount of the abdominal wall and floating substances in the amniotic fluid, as well as the fetus itself. Accordingly, a fetus region must be distinguished from the 3D ultrasound image in order to obtain an uncovered view of the fetus and to reduce the amount of data. Generally, a method for distinguishing the fetus region from its surrounding region in the 3D ultrasound image includes region-of-interest (ROI) selection, surface extraction and region segmentation to remove the undesired data.

The 3D ultrasound imaging system determines the regions of interest (ROIs) enclosing an object in frames (i.e., 2D slices for forming volume data) and combines such ROIs to form a volume of interest (VOI). When manually selecting the ROI, a user selects the ROI by using editing tools to edit or compute a geometrical shape. On the other hand, an automatic selection of the ROT can be performed when priori properties of the object are known such that a statistical model of the object can be estimated. As for the surface extraction of the fetus, the fetus region can be distinguished from each frame, and the contours of the fetus are extracted and combined.

Further, the ultrasound image of the fetus changes at various stages of pregnancy. That is because the level of the amniotic fluid, the shape of the fetus and the position of the fetus in a womb vary according to the gestation stages. The automatic selection of the ROI, which requires relatively low accuracy, can be performed based on information regarding the level of the amniotic fluid and the shape of the fetus depending on the gestation stages. However, the automatic extraction of the contours, which requires relatively high accuracy, can be more complex and thus may be a challenge.

An ultrasound imaging system and a method for forming an ultrasound image according to the invention are defined in independent claims 1 and 5, respectively.

## BRIEF DESCRIPTION OF THE DRAWINGS

Arrangements and embodiments may be described in detail with reference to the following drawings in which like reference numerals refer to like elements and wherein:

FIG. 1 is a block diagram illustrating an ultrasound imaging system constructed in accordance with one embodiment of the present invention;

FIG. 2A depicts equally spaced frames forming volume data;

FIG. 2B illustrates five key frames selected from the frames shown in FIG. 2A;

FIG. 3A shows an original key frame;

FIG. 3B shows ROI determined in the key frame shown in FIG. 3A;

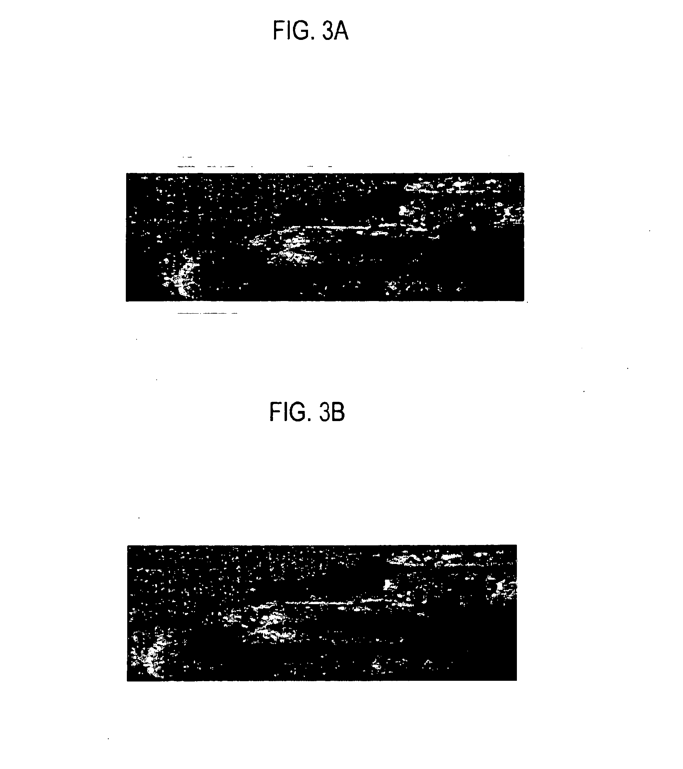

FTG. 3C shows an edge map obtained by performing a LoG operator on the ROI shown in FIG. 3B;

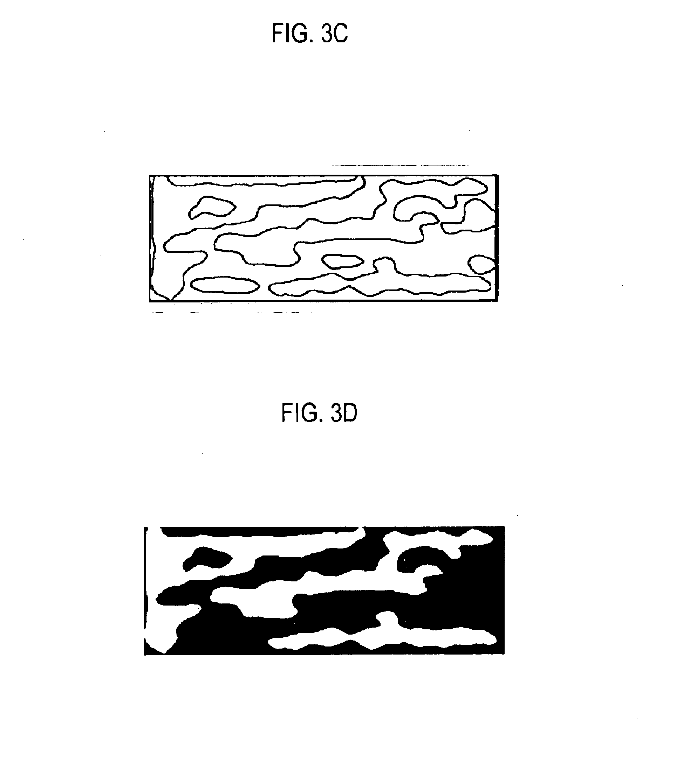

FIG. 3D shows a result of coarse segmentation;

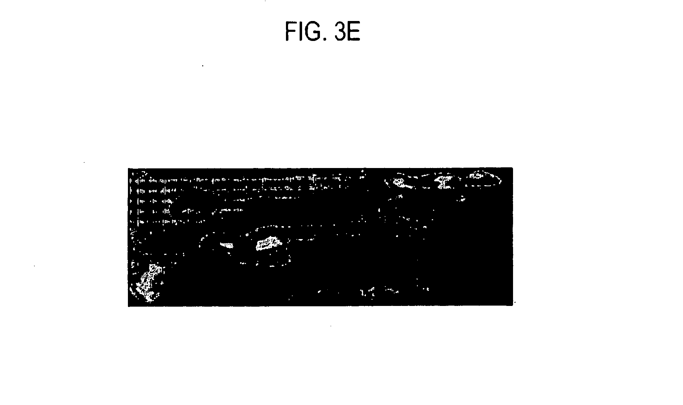

FIG. 3E shows fine-segmented sub-regions, the pixel values of which are approximated by a mean value of intensity;

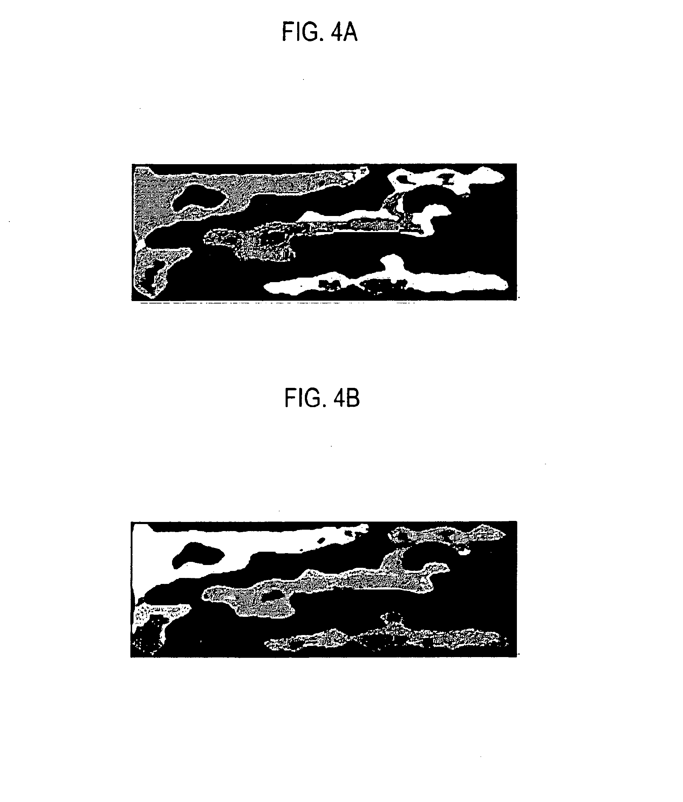

FIGS. 4A and 4B show the results of BDIP and BVLC of the fine-segmented sub-regions, respectively;

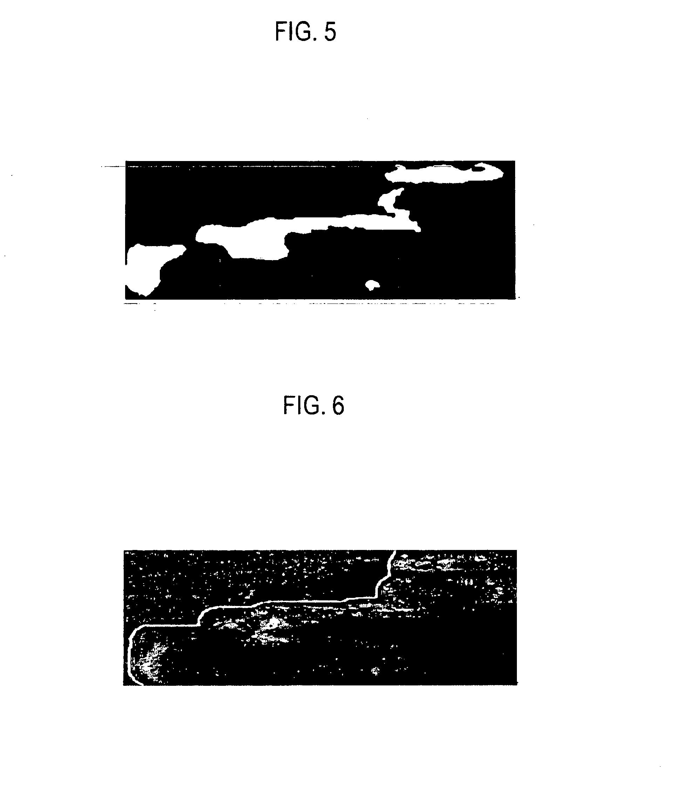

FIG. 5 shows the result obtained by merging fetus regions determined after SVM classification;

FIG. 6 shows an extracted contour of a fetus in a key frame;

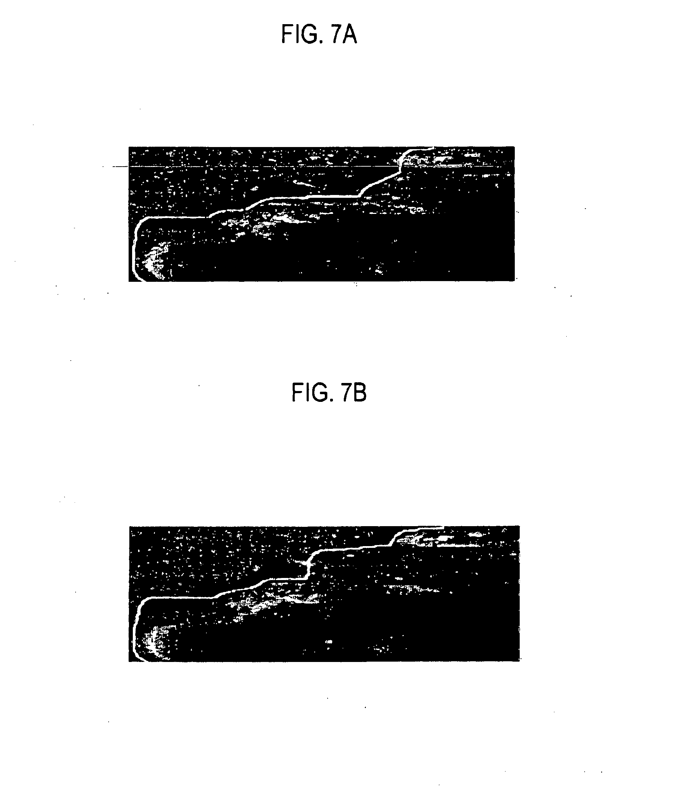

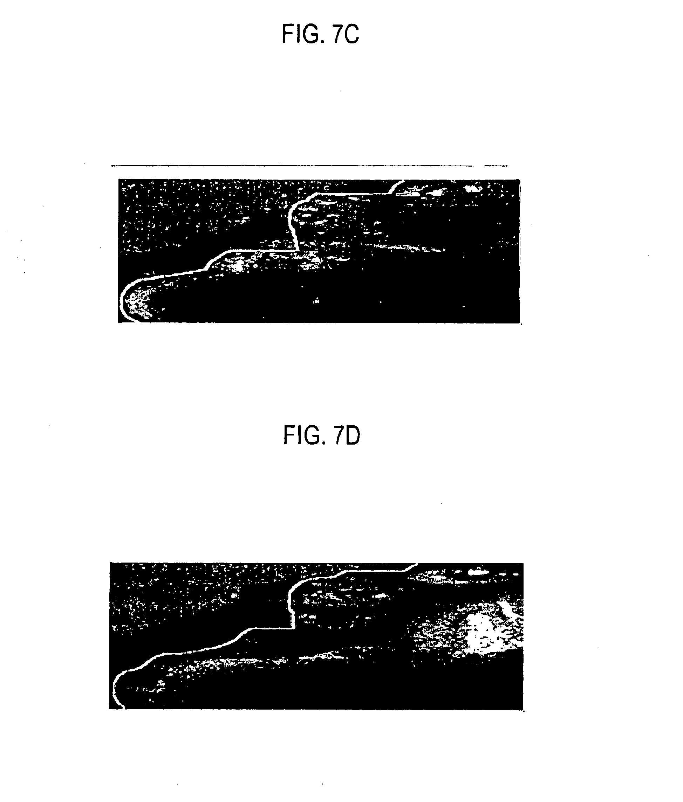

FIGS. 7A to 7D show contours extracted from different key frames;

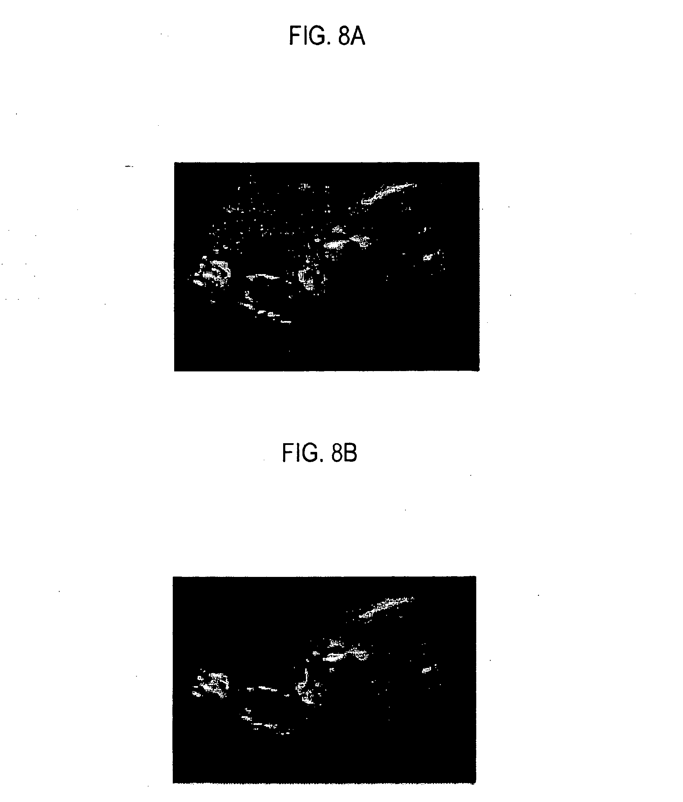

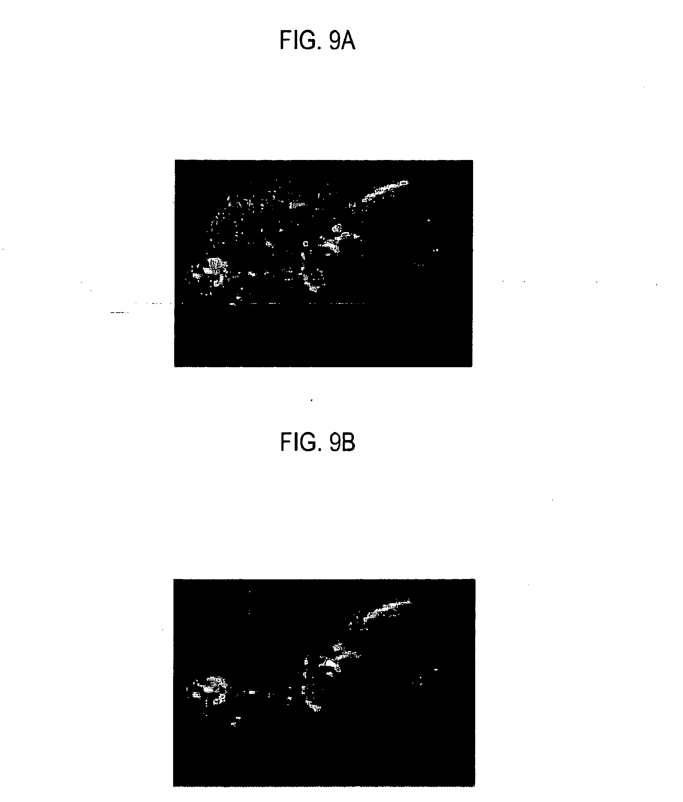

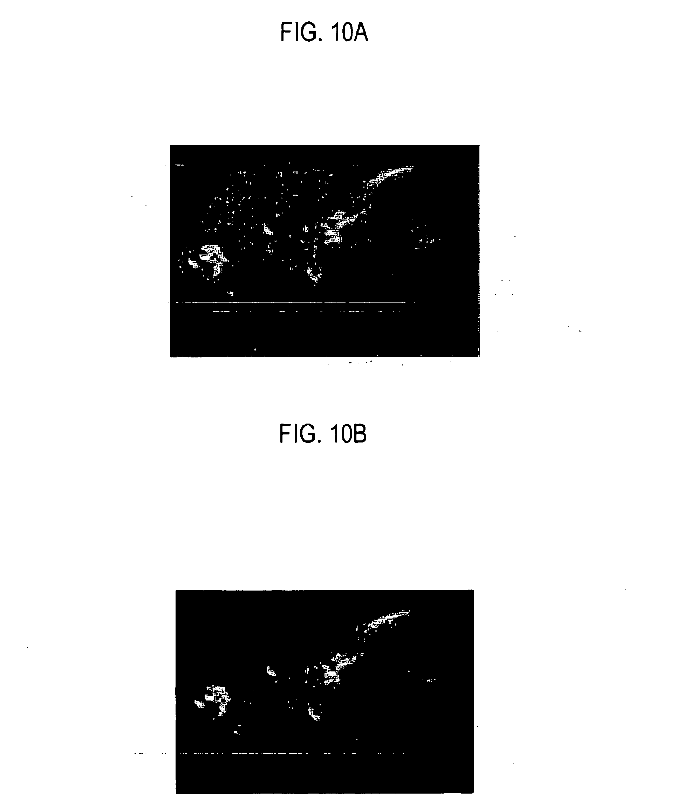

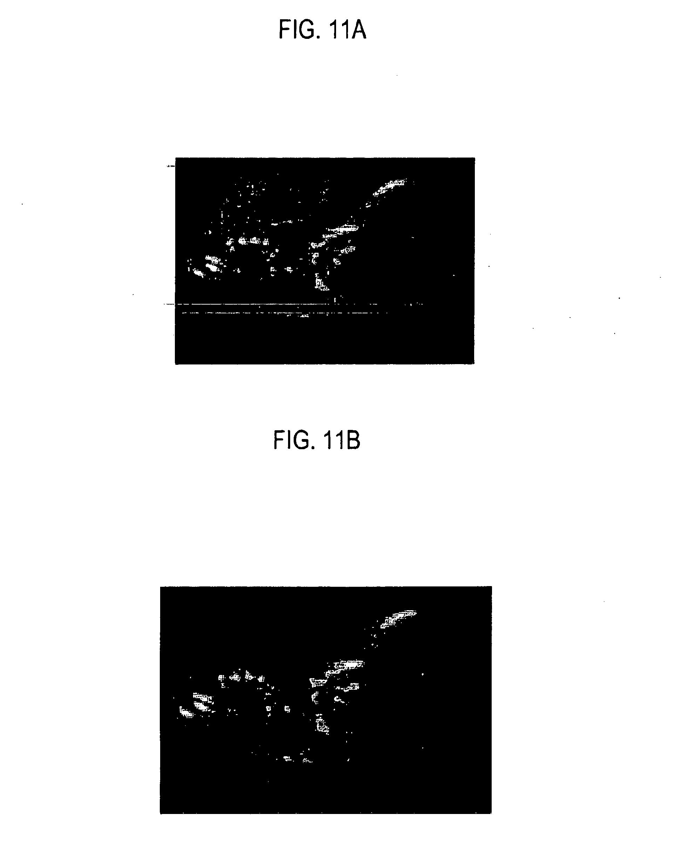

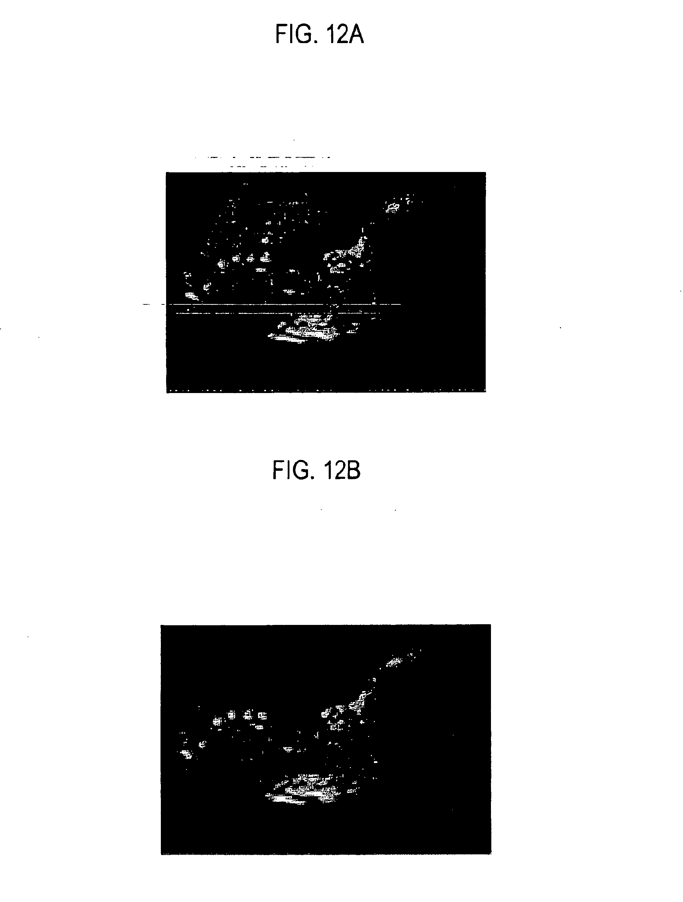

FIGS. 8A, 9A, 10A, 11A and 12A show 2D key frames obtained from original volume data;

FIGS. 8B, 9B, 10B, 11B and 12B show masked volumes formed from the 2D key frames;

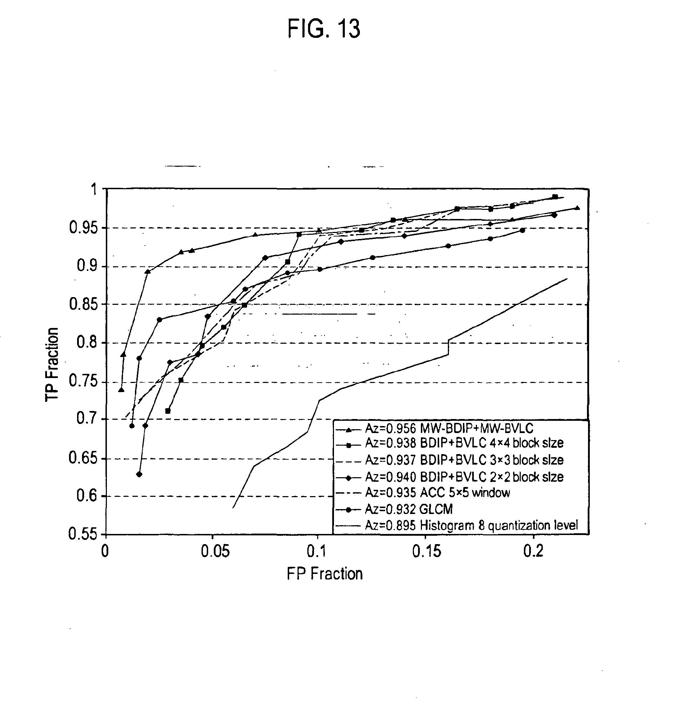

FIG. 13 shows a ROC curve of SVM trained by using feature vectors in accordance with the present invention in comparison with ROC curves of SVM trained by exclusively using BDIP and BVLC;

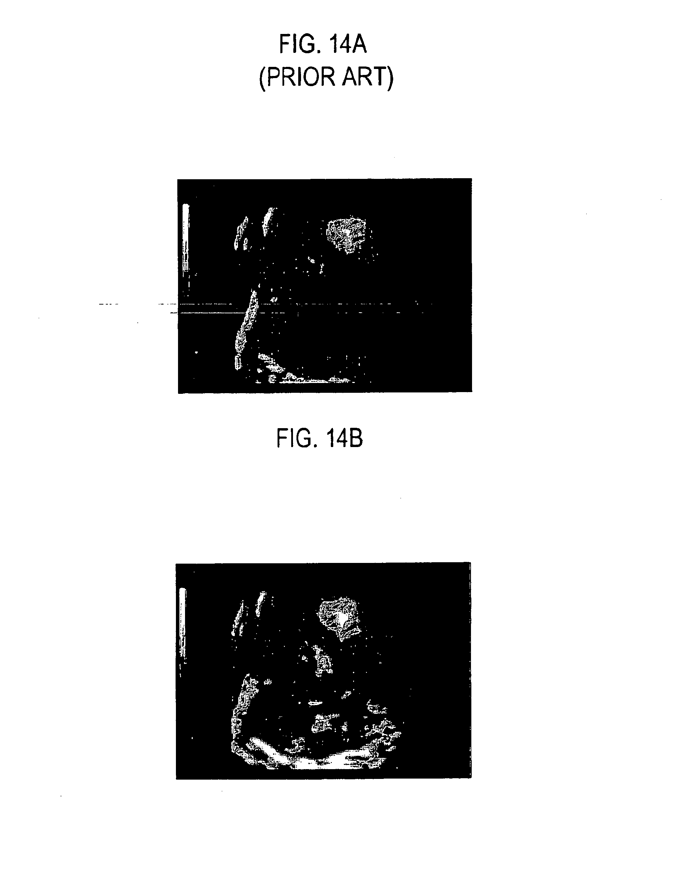

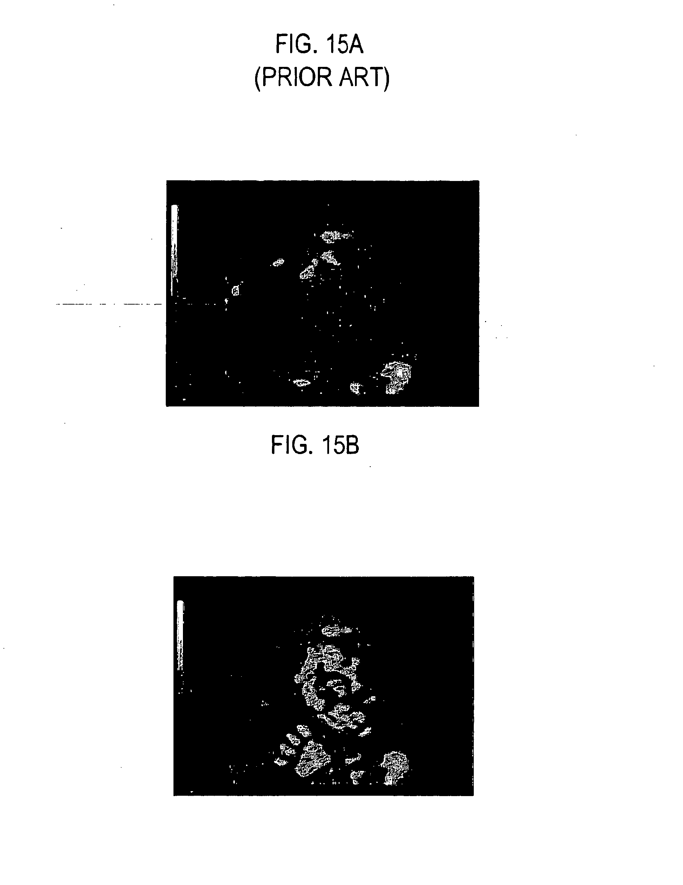

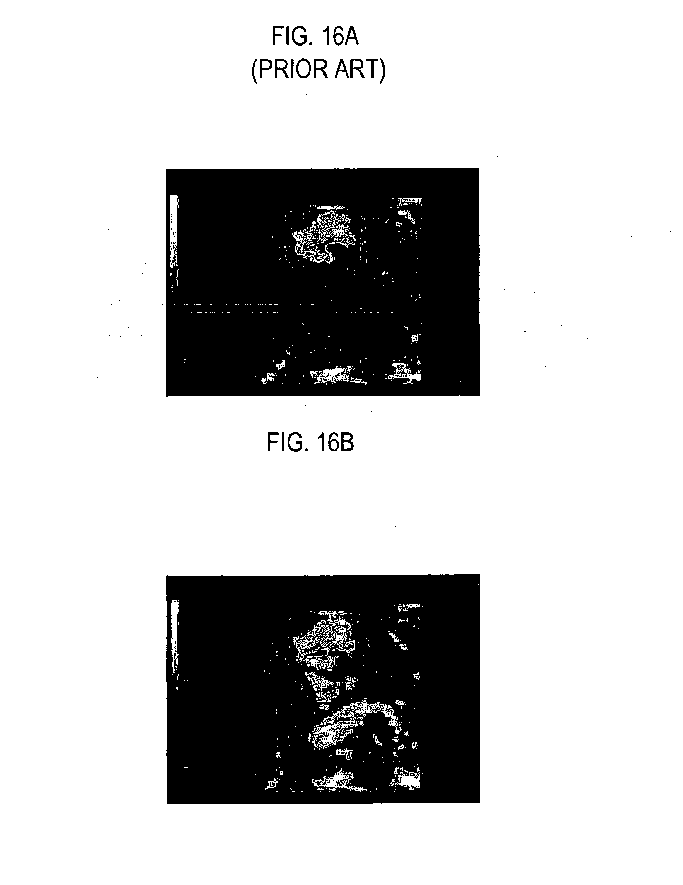

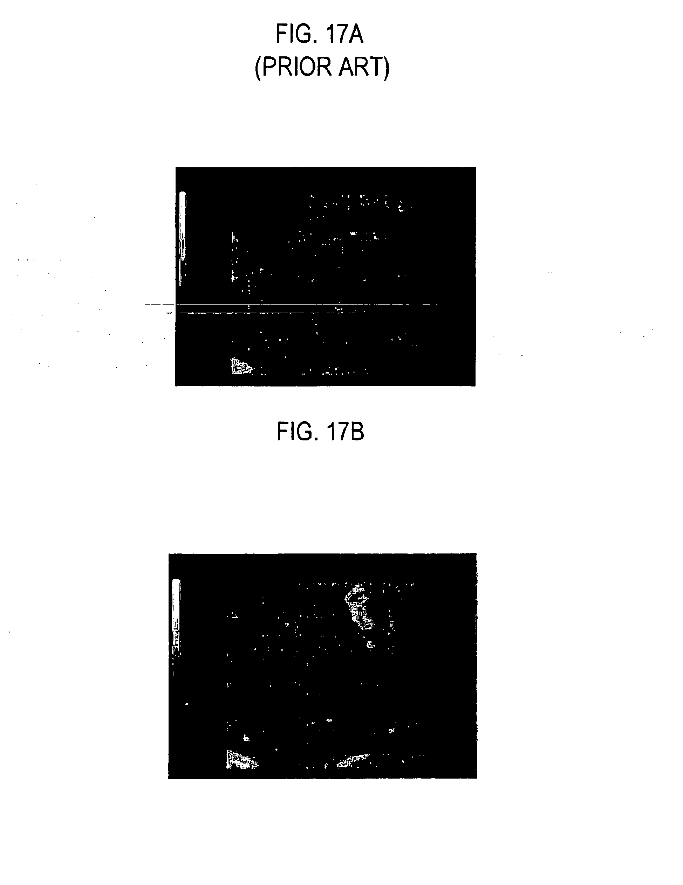

FIGS. 14A, 15A, 16A and 17A are 3D ultrasound photographs of the fetus formed by using a conventional method; and

FIGS. 14B, 15B, 16B and 17B are 3D ultrasound photographs of the fetus formed by using the method of the present invention.

## DETAILED DESCRIPTION

A detailed description may be provided with reference to the accompanying drawings. One of ordinary skill in the art may realize that the following description is illustrative only and is not in any way limiting.

In an embodiment of the present invention, a fetal contour is extracted from 3D volume data without user's operations by using SVM-based texture classification. The fetus floats in a non-fetus region such as amniotic fluid, which is darker than the fetus. Thus, the surface of the fetus can be extracted by removing the dark regions corresponding to the non-fetus region.

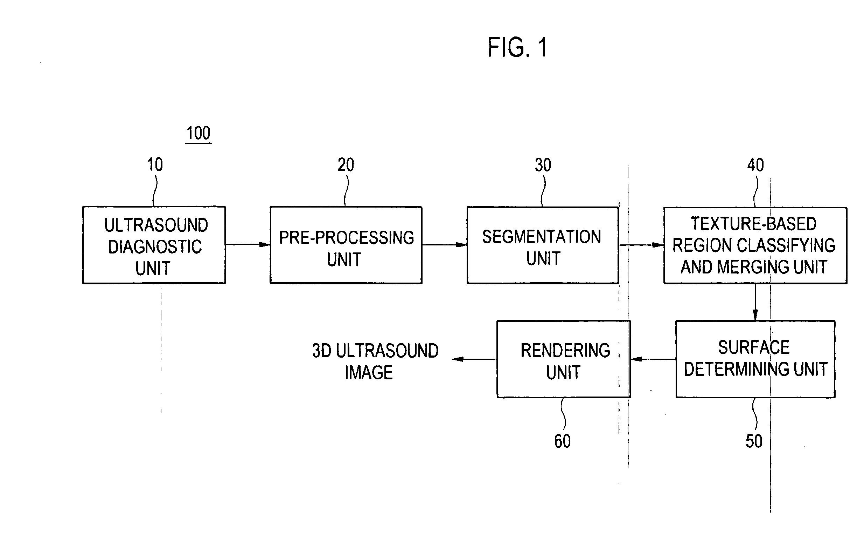

Hereinafter one embodiment of the present invention will be described with reference to the accompanying drawings. FIG. 1 is a block diagram illustrating an ultrasound imaging system constructed in accordance with one embodiment of the present invention.

As shown in FIG. 1, an ultrasound imaging system 100 constructed in accordance with one embodiment of the present invention includes an ultrasound diagnostic unit 10, a pre-processing unit 20, a segmentation unit 30, a texture-based region classifying and merging unit 40, a surface determining unit 50 and a rendering unit 60.

The ultrasound diagnostic unit 10 provides volume data of a target object and its neighboring region. The ultrasound diagnostic unit 10 transmits ultrasound signals to the target object and forms volume data based on receive signals reflected from the target object The volume data is formed with a number of 2D slices, i.e., frames. In an embodiment of the present invention, the target object is a fetus and the neighboring region of the fetus is the abdominal wall of a mother's body, amniotic fluid, floating substances in the amniotic fluid and the like.

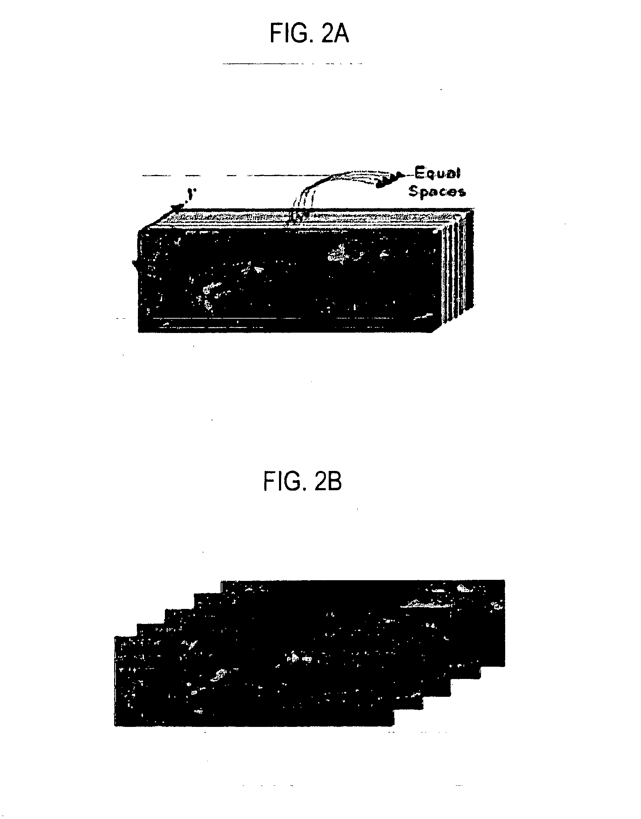

The pre-processing unit 20 selects a predetermined number of key frames from the aforementioned frames. The frames mainly including the information related to the fetus are located at a central portion of the volume data. Further, the frames rarely including such information are located at an edge portion of the volume data. Accordingly, the pre-processing unit 20 selects several frames around a central frame of the frames as key frames. Specifically, two frames located on the left and right of the central frame are selected as boundary frames such that a difference between the average intensity value of the central frame and that of the boundary frame is equal to or greater than a threshold. All frames between the boundary frames including the central frame are selected as the key frames. FIG. 2A shows the frames, which are spaced equally, forming the volume data, whereas FIG. 2B shows five key frames selected from the frames.

The segmentation unit 30 segments each of the key frames into an object region and a non-object region, namely, fetus region and non fetus region. The segmentation unit 30 determines a region of interest (ROI) in the central frame for accurately distinguishing a fetus region from non-fetus region. In such a case, the ROI is determined such that a left boundary of the ROI is located at an amniotic fluid region between the fetus and an abdomen of the mother's body The ROI includes the fetus region and a portion of the non-fetus region, which includes a portion of the amniotic fluid region. The segmentation unit 30 applies the ROI to each of the key frames and performs a Laplacian-of-Gaussian (LoG) operation on the ROI in each key frame, thereby detecting a boundary between the fetus region and the non-fetus region in each ROI. The segmentation unit 30 performs coarse segmentation to remove an amniotic fluid region in the ROI of each key frame on which the LoG operation is performed. The segmentation unit 30 further performs fine segmentation to segment coarse-segmented regions into homogeneous sub-regions.

Generally, in an ultrasound image of the fetus, regions corresponding to the fetus and the abdomen of the mother's body are highly bright regions, which strongly reflect ultrasound pulses. On the other hand, the amniotic fluid region surrounding the fetus is a dark region, which absorbs the ultrasound pulses. Since there is a large difference in intensity between the fetus region and the non-fetus region, the fetus region can be found by detecting a bright region having a clear boundary. The fetus region having the clear boundary is distinguished from the non-fetus region by performing, the LoG operation.

The coarse segmentation is performed to remove a dark region corresponding to the amniotic fluid region in the ROI of each key frame, which includes the fetus region and the non-fetus region distinguished from each other by the LoG operation. In this embodiment, zero-crossing detection is performed as the coarse segmentation to remove the amniotic fluid region (i.e., the dark region) from the key frame. Each key frame is segmented into the non-fetus region (abdomen of the mother's body and floating substances), the fetus region and a mixing region of the fetus region and the non-fetus region. FIG. 3A shows an original key frame and FIG. 3B shows the ROT determined by the left boundary in the key frame shown in FIG. 3A. Further, FIG. 3C shows an edge map obtained by performing the LoG operator on the ROI shown in FIG. 3B, whereas FIG. 3D shows the result of the coarse segmentation.

Fine segmentation is performed to segment the coarse-segmented regions into homogeneous sub-regions. In the ultrasound images, if the abdomen or amniotic fluid overlaps the fetus region, then an unclear edge and speckle may occur in the coarse-segmented regions. The unclear edge and speckle can be removed by using centroid linkage region growing (CLRG), wherein the coarse-segmented regions are finely segmented into homogeneous sub-regions. Accordingly, the fine segmentation leads to more accurate extraction of the fetus region.

In CLRG for segmenting the coarse-segmented regions into homogeneous sub-regions, it is assumed that homogeneity between two regions is measured by a distance between the regions. The distance from a target pixel to its neighboring region is computed by raster scanning. If the distance is short enough, then the target pixel is merged into the neighboring region. Further, if the region merged with a target pixel is close enough to another neighboring region, then the two regions are merged. In this embodiment, the CLRG employs an incremental distortion to measure the distance. In the merging process, the incremental distortion is generated, which is defined by Equation 1.

$$\Delta D = D(R_i \cup R_j) - \left[ D(R_i) + D(R_j) \right] \qquad \text{Eq. 1}$$

In Equation 1, $D(R_i)$ is a sum of squared approximation errors. When the texture of the region is approximated by a mean value, the incremental distortion is expressed by Equation 2.

$$\Delta D = \frac{N_i N_j}{N_i + N_j} (\mu_i - \mu_j)^2 \qquad \text{Eq. 2}$$

In Equation 2, $N_i$ and $N_j$ stand for the number of pixels in $i^{th}$ and $j^{th}$ fine-segmented sub-regions $R_i$ and $R_j$, whereas $\mu_i$ and $\mu_j$ stand for mean values of intensities in the regions $R_i$ and $R_j$.

The threshold $T_i$ for measuring homogeneity is determined for each coarse-segmented region to prevent the boundary of each coarse-segmented region from intersecting a weak edge such as unclear edge and speckle. The threshold $T_i$ is represented by Equation 3.

$$T_i = \mu_i - c \cdot \sigma_i \qquad \text{Eq. 3}$$

In Equation 3, $\mu_i$ and $\sigma_i$ represent a mean value and a standard deviation of intensity in the fine-segmented sub-region $R_i$, while $c$ represents a constant. FIG. 3E shows the fine-segmented sub-regions, the pixel values of which are approximated by the mean value of intensity.

The texture-based region classifying and merging unit 40 classifies the fine-segmented sub-regions into fetus sub-regions and non-fetus sub-felons based on the texture. It then forms a fetus image by removing the non-fetus sub-regions and merging the fetus sub-regions in each key frame. When the fetus and abdomen legions are merged, there occurs a problem in classifying the sub-regions since the fetus region and the abdomen region have a similar mean intensity. However, since fetus bones and tissues reflect ultrasound pulses at different reflection levels, the fetus and abdomen regions have different intensity variations and texture smoothness. By using block difference inverse probabilities (BDTP) and block variation of local correlation coefficients (BVLC), which are known to be efficient in image retrieval, the sub-regions may be classified into the fetus sub region and abdomen sub-region (non-fetus sub-region). To increase the accuracy, the texture of the sub-regions can be measured by using multi-window BDIP and BVLC moments.

BDIP and BVLC will now be described below.

BDTP measures the intensity variation in a block in a frame on which the fine segmentation is performed. BDIP is defined as the ratio of a sum of values, which are obtained by subtracting the pixel values in the block from the maximum pixel value in the block to the maximum pixel value in the block. Generally, BDIP of a block having the size $W$ x W can be defined by Equation 4.

$$\beta^k(l) = \frac{\frac{1}{W^2} \sum_{(i,j) \in B_l^k} \left[ \max_{(i,j) \in B_l^k} I(i,j) - I(i,j) \right]}{\max_{(i,j) \in B_l^k} I(i,j)} \qquad \text{Eq. 4}$$

In Equation 4, $I(i,j)$ denotes intensity of a pixel $(i,j)$ in the block and $l = (u,v)$, a location of the block in the image $k$ is defined as a maximum distance between two pixels of a pixel pair in the block and is equivalent to $W$-1. The BDIP increases in proportion to the intensity variation in the block.

BVLC is defined as a variation of local correlation coefficients (LCCs) in a block having the size $WxW$ according to four directions (-90°, 0°, -45° and 45°). LCC in each direction can be represented by Equation 5.

$$\rho^k(l) = \frac{\frac{1}{W^2} \sum_{(i,j) \in B_l^k} I(i,j) I(i+\Delta_i(k), j+\Delta_j(k)) - \mu_l \mu_{l+\Delta(k)}}{\sigma_l \sigma_{l+\Delta(k)}} \qquad \text{Eq. 5}$$

In Equation 5, $l = (u,v)$ denotes the location of the block in the image, whereas $\mu_l$ and $\sigma_l$ represent the local mean value and the local standard deviation of intensity in the block, respectively. $\Delta(k) = (\Delta_i(k), \Delta_j(k))$ represents the position variance of the block moving in four directions. Accordingly, $\mu_{l+\Delta(k)}$ and $\sigma_{l+\Delta(k)}$ represent a mean value and a standard deviation of the block that is moved by $\Delta(k)$, respectively. Thus, a value of BVLC can be expressed by Equation 6.

$$\gamma^k(l) = \max_{\Delta(k) \in O_4} [\rho^k(l)] - \min_{\Delta(k) \in O_4} [\rho^k(l)],$$

$$\text{Eq. 6}$$

$$O_4 = \{(0,k), (k,0), (k,k), (k,-k)\}$$

From Equation 6, it can be seen that the larger the roughness in the block, the higher the value of BVLC.

In order to better utilize the texture features of the fetus and non-fetus regions, BDIP and BVLC moments of various window sizes are calculated and combined to produce multi-window BDIP (MW-BDIP) and multi-BVLC (MW-BVLC) moments. It is known that the MW-BDIP and MW-BVLC moments have efficient texture features in image retrieval. In this embodiment, the sub-regions are classified into the fetus and non-fetus regions by using SVM. That is, the feature vectors of SVM are obtained based on the MW-BDIP and MW-RVLC moments, while the intensity variation and smoothness of tissues in the fetus and abdomen regions are characterized by using the feature vectors.

Each key frame is segmented into non-overlapping blocks of various sizes. Then, the BDIP and BVLC moments arc computed in each block in a similar way as in the ROI determination. The only difference between the calculation of BDIP and BVLC moments and ROT determination is that the thresholds in block classification for calculation of BDIP and BVLC moments are determined for each segmented region. The feature vector $\mathbf{x}^k$ for a window having the size $(k+1)^2$ is defined as a combination of BDIP and BVLC moments, as shown by Equation 7.

$$\mathbf{x}^k = \left[\mu_1^k(D), \mu_2^k(D), ..., \mu_n^k(D), \mu_1^k(V), \mu_2^k(V), ..., \mu_n^k(V)\right], \quad k = 1, 2, 3 \quad \text{Eq. 7}$$

In Equation 7, $\mu_i^k(D)$ and $\mu_i^k(V)$ denote BDIP and BVLC means for the $i^{th}$ class for each $k$, respectively. The feature vectors $x^k (k=1, 2, 3)$ arc computed and the MW feature vectors for SVM arc obtained by the combination of the feature vectors. In order to prevent a feature vector with a large variation in SVM calculation from becoming dominant, the components of the feature vector are normalized based on the variations computed from training database (DB), as shown by Equation 8.

$$\mathbf{x} = [\frac{\mathbf{x}^1}{\sigma^1} \quad \frac{\mathbf{x}^2}{\sigma^2} \quad \frac{\mathbf{x}^3}{\sigma^3}] \qquad \text{Eq. 8}$$

In Equation 8, component-wise division is performed. Further $\sigma^k$ represents the standard deviations calculated from the training DB for each $k$.

The results of BDIP and BVLC of the fine-segmented sub-regions for $k=1$ are shown in FIGS. 4A and 4B, respectively. The results shown in FIGS. 4A and 4B are obtained by increasing the dynamic range and equalizing the histogram to discriminate the intensity differences of the sub-regions.

When each feature vector $x_i$ ($x_i \in R^N$) belongs to a class $y_i$ ($y_i \in (\pm 1)$), a training set of examples is referred to as $\{(x_i, y_i), i =1, ... , N\}$. A SVM classifier is based on a hyperplane for distinguishing the classes of +1 and -1, while the distance between each class and the hyperplane is maximized. $\Lambda$ constrained second-order optimization problem is solved by introducing Lagrange multipliers $\alpha_i$ (i=1, 2, $\cdots$, N) so that a unique optimal hyperplane can be obtained. In such a case, solutions of the constrained second-order optimisation problem can be expressed by a subset of support vectors. Linearly separable data can be obtained by analyzing the hyperplane, whereas non-lincarly separable data that are generally generated in substantial separation problem can be analyzed by using an adequate non-linear operator $\Phi(\cdot)$ for mapping input feature vectors in higher-order feature vector space. A new sample x can be classified by using a non-linear classifier, as shown by Equation 9 below.

$$f(\mathbf{x}) = \text{sgn}\left(\sum_{i=1}^{N} \alpha_i y_i \Phi(\mathbf{x}_i)\Phi(\mathbf{x}) + b\right) = \text{sgn}\left(\sum_{i=1}^{N} \alpha_i y_i \mathbf{K}(\mathbf{x}_i, \mathbf{x}) + b\right) \qquad \text{Eq. 9}$$

In Equation 9, K stands for a kernel function satisfying the Mercer's theorem. The kernel function generally used is polynomials, Gaussian radial basic function (RBF) or tag-sigmoid. In this embodiment, an SVM classifier adopting Gaussian RBP non linear kernel, which is relatively efficient compared to other. SVM classifiers, is employed to classify the fine-segmented sub-regions.

As described above, a fetus image is formed by removing the non-fetus regions from the classified sub-regions and merging the fetus regions adjacent to each other in each key frame. FIG. 5 shows the result obtained by merging the regions determined as the fetus regions after SVM classification.

The surface determining unit 50 extracts the fetal contour in each key frame and determines a 3D surface of the fetus by connecting the extracted fetal contours. The fetal contour is extracted by finding a first pixel in each horizontal search line belonging to the surface of the fetus. FTG. 6 shows an extracted contour of the fetus in a first key frame, whereas FIGS. 7A to 7D show the contours extracted from the other key frames.

The rendering unit 60 forms a masked volume and produces a 3D image of the fetus by rendering the masked volume. The masked volume is formed only with the 3D surface in which the abdomen region of the mother's body and the floating substances are removed.

The 3D image of the fetus can be formed by using the fetus contours extracted from the key frames in consideration of spatial continuity of the fetus surface. The contours extracted from the two neighboring key frames are used for linear intrapolation of all frames inserted between the two key frames. The contours extracted from the other two frames at both ends are used to extrapolate the frames outside the two key frames. Since the intrapolated frames and the extrapolated frames contain relatively less visual information, they do not affect the quality of the image. The masked volume data are formed from the determined 3D surface while it is assumed that there is no data outside the 3D surface. FIGS. 8A, 9A, 10A, 11A and 12A show 2D key frames obtained from original volume data, whereas FIGS. 8B, 9B, 10B, 11B and 12B show the masked volumes formed from the 2D key frames.

An experimental result will now be described below,

1. Settings for experimentation

Forty sets of raw volume data with various sizes at different gestation stages are prepared to evaluate the effects of automatic surface extraction in accordance with the present invention. A number of frames are extracted from each volume data in the sagittal direction, while 10 frames around a central frame are selected from the frames of each volume data to obtain a total of 400 frames.

In each of the 400 frames, two non-overlapping regions of size $M$ x $M$ are selected in the fetus region and the abdomen region of the mother's body, respectively. The sizes of the non-overlapping regions should be determined such that the non-overlapping regions do not contain a portion of the abdomen and floating substances in the frames. In this experiment, it is determined that M is 32. The selected 1600 regions are divided into two groups, each of which includes 400 fetus regions and 400 non-fetus regions.

In coarse segmentation, the LoG operator is performed with a standard deviation $\sigma$ of 9, which shows the most excellent segmentation result in the training of different $\sigma$ values. In order to remove the small regions, regions of sizes smaller than a threshold $T_{SIZR}$ =100 are removed.

When calculating the BDIP and BVLC, the block size is chosen as 2 x 2. When selecting the SVM classifier adopting Gaussian RBF kernel, a grid search for the best parameters of width w and regularization factor C is performed by a four-fold cross-validation procedure on the training set With the most accurate cross-validation, the SVM classifier with $\sigma$=2 and C=15 is used for region classification. The number of classes for block classification in the calculation of BDIP and BVLC moments is determined to be 4.

2. Performance evaluation

The receiver operating characteristic (ROC) analysis is used for providing a comprehensive summary of the trade-off between the sensitivity and specificity of the SVM. To investigate the contribution of BDIP and BVLC moments to the role of the proposed feature vectors, the ROC curve of the SVM trained by using the feature vectors is compared with the ROC curves of the SVM trained by exclusively using BDIP and BVLC. As shown in FIG. 13, the combination of BDIP and BVLC yields the highest SVM performance (curves under the curve $A_3$ = 0.94).

Table 1 represents the performance of the SVM classifier evaluated for 800 testing sets containing the fetus and non-fetus regions, wherein the accuracy, sensitivity and specificity arc 93.38 %, 95.5 % and 91.25 %, respectively. Further, when the SVM classifier is applied to 400 key frames, the number of correctly extracted contours is visually observed as 368/400 (about 92%).

Table 1

| Test set : 800 | Accuracy | Sensitivity | Specificity |
|---|---|---|---|
| SVM performance | 747/800 (93.38%) | 382/400(95.5%) | 365/400(91.25%) |

3. Visualization

Visualization is based on the ray-casting method to form the 3D image by directly processing the VOI without visual expression. Since the data defined by the masked volume is only rendered, the system can display the ultrasound images in real time. FIGS. 14A, 15A, 16A and 17A are 3D ultrasound photographs of the fetus formed by using the conventional method, whereas FIGS. 14B, 15B, 16B and 17B are 3D ultrasound photographs of the fetus formed by using the method in accordance with the present invention.

An embodiment may be achieved in whole or in part by an ultrasound imaging system, which includes: an ultrasound diagnostic unit for providing 3D volume data of an object and its neighboring region, wherein the 3D volume data is formed with a number of frames; a pre-processing unit for selecting a predetermined number of key frames from the frames; a segmentation unit for segmenting each of the key frames into object regions and non-object regions; a texture-based region classifying and merging unit for classifying the object regions and the non-object regions into object sub-regions and non-object sub-regions based on the texture thereof, the texture-based region classifying and merging unit further being configured to remove the non-object sub-regions in the key frames and merge the object sub-regions; a surface determining unit for extracting contours of the object in the key frames and determining a 3D surface of the object by connecting the contours extracted from the key frames; and a rendering unit for forming masked volume with the determined 3D surface and forming a 3D ultrasound image of the object by rendering the masked volume.

Any reference in this specification to "one embodiment," "an "embodiment," "example embodiment," etc., means that a particular feature, structure or characteristic described in connection with the embodiment is included in at least one embodiment of the invention. The appearances of such phrases in various places in the specification are not necessarily all referring to the same embodiment.

Although embodiments have been described with reference to a number of illustrative embodiments thereof, it should be understood that numerous other modifications and embodiments can be devised by those skilled in the art that will fall within the scope of the appended claims. More particularly, numerous variations and modifications are possible in the component parts and/or arrangements of the subject combination arrangement within the scope of the disclosure, the drawings and the appended claims.

**Claims**

1. An ultrasound imaging system (100), comprising:

   an ultrasound diagnostic unit (10) for providing 3D volume data of a fetus and a neighboring region of the fetus, wherein the 3D volume data is formed with a number of frames;
   a pre-processing unit (20) for selecting a predetermined number of frames around a central frame from the frames as key frames;
   a segmentation unit (30) for segmenting each of the key frames into fetus regions and non-fetus regions;
   a texture-based region classifying and merging unit (40) for classifying the fetus regions and the non-fetus regions into fetus sub-regions and non-fetus subregions based on the texture thereof, the texture-based region classifying and merging unit further being configured to remove the non-fetus sub-regions in the key frames and merge the fetus sub-regions;
   a surface determining unit (50) for extracting contours of the fetus in the key frames with the non-fetus subregions removed and determining a 3D surface of the fetus by connecting the contours extracted from the key frames; and
   a rendering unit (60) for forming masked volume with the determined 3D surface and forming a 3D ultrasound image of the fetus by rendering the masked volume.

2. The ultrasound imaging system of Claim 1, wherein non-fetus regions include an amniotic fluid region and an abdomen region of a mother's body.

3. The ultrasound imaging system of Claim 2, wherein the segmentation unit (30) is configured to determine a region of interest ROT in one of the key frames and to apply the ROI to each of the key frames, the segmentation unit being configured to segment the ROI in each of the key frames into the fetus regions and the non-fetus regions by performing a Laplacian-of Gaussian LoG operation and perform coarse segmentation on the ROI that underwent the LoG operation to form coarse-segmented regions and to remove the amniotic fluid region in the ROI, the segmentation unit (30) further being configured to segment the coarse-segmented regions into homogeneous subregions obtained by merging neighboring coarse-segmented regions based on a distance therebetween by performing fine segmentation.

4. The ultrasound imaging system of Claim 3, wherein the texture-based region classifying and merging unit (40) is configured to segment each of the key frames into non-overlapping blocks of various sizes and to compure block difference inverse probabilities BDIP and block variation of local correlation coefficients BVLC moments in each block to measure texture of the sub-regions obtained by the fine segmentation, the texture-based region classifying and merging unit (40) further being configured to classify the sub-regions into fetus sub-regions and non-fetus subregions by using a support vector machine SVM, wherein BDIP is defined as the ratio of a sum of values, which are obtained by subtracting the pixel values in the block from the maximum pixel value in the block, to the maximum pixel value in the block, and BVLC is defined as a variation of local correlation coefficients in the block according to four directions -90°, 0°, -45° and 45°.

5. A method for forming an ultrasound image, comprising:

   a) providing 3D volume data of a fetus and a neighboring region of the fetus, wherein the 3D volume data is formed with a number of frames;

b) selecting a predetermined number of frames around a central frame from the frames as key frames;

c) segmenting each of the key frames into fetus regions and non-fetus regions;

d) classifying the fetus regions and the non-fetus regions into fetus sub-regions and non-fetus sub-regions based on texture thereof, and removing the non-fetus sub-region in each of the key frames and merging the fetus sub-regions;

e) extracting contours of the fetus in the key frames with the non-fetus sub-regions removed and determining a 3D surface of the fetus by connecting the contours extracted from the key frames; and fetus by connecting the

f) forming masked volume with the determined 3D surface and forming a 3D ultrasound image of the fetus by rendering the masked volume.

6. The method of Claim 5, wherein non-fetus regions include an amniotic fluid region and an abdomen region of a mother's body.

7. The method of Claim 6, wherein the step c) includes:

determining a region of interest ROI in one of the key frames;

applying the ROI to each of the key frames;

segmenting the ROI in each of the key frames into the fetus regions and the non-fetus regions by performing a Laplacian-of-Gaussian LoG operation;

performing coarse segmentation on the ROI that underwent the LoGi operation to form coarse-segmented regions and to remove the amniotic fluid region in the ROI; and

segmenting the coarse-segmented regions into homogeneous sub-regions obtained by merging neighboring coarse-segmented regions based on a distance therebetween by performing fine segmentation.

8. The method of Claim 7, wherein the step d) includes:

segmenting each of the key frames into non-overlapping blocks of various sizes;

computing block difference inverse probabilities BDIP and block variation of local correlation coefficients BVLC moments in each block to measure texture of the sub-regions obtained by the fine segmentation, wherein BDIP is defined as the ratio of a sum of values, which are obtained by subtracting the pixel values in the block from the maximum pixel value in the block, to the maximum pixel value in the block, and BVLC is defined as a variation of local correlation coefficients in the block according to four directions -90°, 0°, -45° and 45°; and

classifying the sub-regions into fetus sub-regions and non-fetus sub-regions by using a support vector machine SVM.

**Patentansprüche**

1. Ultraschallabbildungssystem (100), welches Folgendes aufweist:

eine Ultraschall-Diagnostizereinheit (10) zum Erzeugen von 3D-Volumendaten eines Fötus und einer Nachbarregion des Fötus, wobei die 3D-Volumendaten mit einer Vielzahl von Rahmen gebildet werden;

eine Vorverarbeitungseinheit (20) zum Auswählen einer vorbestimmten Anzahl von Rahmen, um einen zentralen Rahmen aus den Rahmen als Keyframe bzw. Schlüsselrahmen auszuwählen;

eine Zerlegungseinheit (30) zum Zerlegen von jedem der Keyframes in Fötus-Regionen und Nicht-Fötus-Regionen;

eine strukturbasierte Regionen-Klassifizierungs- und Verbindungseinheit (40) zum Klassifizieren der Fötus-Regionen und der Nicht-Fötus-Regionen in Fötus-Unterregionen und Nicht-Fötus-Unterregionen basierend auf der Struktur derselben, wobei die strukturbasierte Regionen-Klassifizierungs- und Verbindungseinheit (40) des Weiteren dafür vorgesehen ist, die Nicht-Fötus-Unterregionen in den Keyframes zu entfernen und die Fötus-Unterregionen zu verbinden;

eine Oberflächenermittlungseinheit (50) zum Extrahieren von Konturen des Fötus in die Keyframe, wobei die Nicht-Fötus-Unterregionen entfernt sind, und zum Ermitteln bzw. festlegen einer 3D-Oberfläche des Fötus durch Verbinden der aus den Keyframes extrahierten Konturen; und

eine Rendereinheit (60) zum Erzeugen eines abgedeckten Volumens mit der ermittelten 3D-Oberfläche und zum Erzeugen eines 3D-Ultraschallbilds des Fötus durch Rendern des abgedeckten Volumens.

2. Ultraschallabbildungssystem nach Anspruch 1, wobei die Nicht-Fötus-Regionen eine Fruchtwasserregion und eine Bauchregion eines Körpers einer Mutter beinhalten.

3. Ultraschallabbildungssystem nach Anspruch 2, wobei die Zerlegungseinheit (30) dafür vorgesehen ist, einen interessierenden Bereich ROI in einem der Keyframes zu ermitteln und den ROI auf jeden der Keyframes anzuwenden, wobei die Zerlegungseinheit des Weiteren dafür vorgesehen ist, den ROI in jedem der Keyframes in die Fötus-Regionen und die Nicht-Fötus-Regionen mittels Durchführen einer Laplacian of Gaussian LOG-Operation zu zerlegen und eine grobe Zerlegung an dem ROI, der der LOG-Operation unterzogen wurde, durchzuführen, um grob zerlegte Regionen zu bilden und um die Fruchtwasserregion in dem ROI zu entfernen, wobei die Zerlegungseinheit (30) des Weiteren dafür vorgesehen ist, die grob zerlegten Regionen in homogene Unterregionen zu zerlegen, die durch Verbinden benachbarter, grob zerlegter Regionen basierend auf einem Abstand zwischen denselben mittels Durchführen einer feinen Zerlegung erhalten wurden.

4. Ultraschallabbildungssystem nach Anspruch 3, wobei die strukturbasierte Regionen-Klassifizierungs- und Verbindungseinheit (40) dafür vorgesehen ist, jeden der Keyframes in nicht überlappende Blöcke von unterschiedlichen Größen zu zerlegen und Blockdifferenz-Invers-Wahrscheinlichkeiten BDIP und Blockvariationen von lokalen Korrelationskoeffizienten BVLC-Momente in jedem Block zu berechnen, um die Struktur der durch die feine Zerlegung erhaltenen Unterregionen zu messen, wobei die strukturbasierte Regionen-Klassifizierungs- und Verbindungseinheit (40) des Weiteren dafür vorgesehen ist, die Unterregionen (in Fötus-Unterregionen und Nicht-Fötus-Unterregionen) unter Verwendung einer Support Vector Machine SVM zu klassifizieren, wobei BDIP als das Verhältnis einer Summe von Werten definiert ist, welche durch Subtrahieren der Pixelwerte in dem Block von dem maximalen Pixelwert in dem Block zu dem maximalen Pixelwert in dem Block erhalten werden, und wobei BVLC als eine Variation von lokalen Korrelationskoeffizienten in dem Block gemäß vier Richtungen -90°, 0 °, -45° und 45° definiert ist.

5. Verfahren zum Erzeugen eines Ultraschallbilds, welches Folgendes aufweist:

   a) zur Verfügung Stellen von 3D-Volumendaten eines Fötus und einer benachbarten Region des Fötus, wobei die 3D-Volumendaten mit einer Vielzahl von Rahmen gebildet werden;
   b) Auswählen einer vorbestimmten Anzahl von Rahmen, um einen zentralen Rahmen von den Rahmen als Keyframe auszuwählen;
   c) Zerlegen von jedem der Keyframes in Fötus-Regionen und Nicht-Fötus-Regionen;
   d) Klassifizieren der Fötus-Regionen und der Nicht-Fötus-Regionen in Fötus-Unterregionen und Nicht-Fötus-Unterregionen basierend auf der Struktur derselben und Entfernen der Nicht-Fötus-Unterregionen in jedem der Keyframes und Verbinden der Fötus-Unterregionen;
   e) Extrahieren von Konturen des Fötus in den Keyframes, wobei die Nicht-Fötus-Unterregionen entfernt sind, und Ermitteln bzw. Festlegen einer 3D-Oberfläche des Fötus durch Verbinden der aus den Keyframes extrahierten Konturen; und
   f) Erzeugen eines abgedeckten Volumens mit der ermittelten 3D-Oberfläche und Erzeugen eines 3D-Ultraschallbilds des Fötus durch Rendern des abgedeckten Volumens.

6. Verfahren nach Anspruch 5, wobei die Nicht-Fötus-Regionen eine Fruchtwasserregion und eine Bauchregion eines Körpers einer Mutter beinhalten.

7. Verfahren nach Anspruch 6, wobei der Schritt c) Folgendes aufweist:

   Ermitteln eines interessierenden Bereichs ROI in einem der Keyframes;
   Anwenden des ROI auf jeden der Keyframes;
   Zerlegen des ROI in jedem der Keyframes in die Fötus-Regionen und die Nicht-Fötus-Regionen mittels Durchführen einer Laplacian of Gaussian LOG-Operation;
   Durchführen einer groben Zerlegung an dem ROI, der der LOG-Operation unterzogen wurde, um grob zerlegte Regionen zu bilden und um die Fruchtwasserregion in dem ROI zu entfernen; und
   Zerlegen der grob zerlegten Regionen in homogene Unterregionen, die durch Verbinden benachbarter grob zerlegter Regionen basierend auf einem Abstand zwischen denselben mittels Durchführen einer feinen Zerlegung erhalten wurden.

8. Verfahren nach Anspruch 7, wobei der Schritt d) Folgendes aufweist:

   Zerlegen von jedem der Keyframes in nicht überlappende Blöcke von unterschiedlichen Größen;
   Berechnen von Blockdifferenz-Invers-Wahrscheinlichkeiten BDIP und Blockvariationen von lokalen Korrelationskoeffizienten BVLC-Momenten in jedem Block, um die Struktur der durch die feine Zerlegung erhaltenen Unterregionen zu messen, wobei BDIP als das Verhältnis einer Summe von Werten definiert ist, welche durch Subtrahieren der Pixelwerte in dem Block von dem maximalen Pixelwert in dem Block zu dem maximalen Pixelwert in dem Block erhalten werden, und wobei BVLC als eine Variation von lokalen Korrelationskoeffizienten in dem Block gemäß vier Richtungen -90°, 0°, -45° und 45° definiert ist; und

Klassifizieren der Unterregionen in Fötus-Unterregionen und Nicht-Fötus-Unterregionen unter Verwendung einer Support Vector Machine SVM.

**Revendications**

1. Système d'imagerie ultrasonique (100) comportant :

   une unité ultrasonique de diagnostic (10) pour fournir des données de volume en 3D d'un foetus et d'une région voisine du foetus, dans lequel les données de volume en 3D sont constituées d'un nombre de trames ;
   une unité de prétraitement (20) pour sélectionner un nombre prédéterminé de trames autour d'une trame centrale parmi les keyframes;
   une unité (30) de segmentation pour segmenter chacune des keyframes en régions de foetus et en régions sans foetus ;
   une unité (40) de classification et de groupage basée sur la texture locale pour classifier les régions à foetus et les régions sans foetus en sous-régions à foetus et en sous-régions sans foetus, sur la base de leur texture, l'unité de classification et de groupage basée sur la texture locale étant en outre configurée pour supprimer les sous-régions sans foetus dans les keyframes et grouper les sous-régions à foetus ;
   une surface de détermination (50) pour extraire les contours de foetus dans les keyframes, les sous-régions sans foetus étant supprimées et pour déterminer une surface 3D du foetus en connectant les contours extraits des keyframes; et
   une unité de restitution (60) pour former un volume masqué avec la surface 3D déterminée et pour former une image ultrasonique en 3D du foetus en restituant le volume masqué.

2. Système d'imagerie ultrasonique selon la revendication 1, dans lequel les régions sans foetus comprennent les régions à fluide amniotique et la région de l'abdomen du corps d'une mère.

3. Système d'imagerie ultrasonique selon la revendication 2, dans lequel l'unité de segmentation (30) est configurée pour déterminer une région présentant un intérêt ROI dans un des keyframes et pour appliquer la ROI pour capturer les keyframes, l'unité de segmentation étant configurée pour segmenter la ROI pour capturer les keyframes dans les régions à foetus et les régions sans foetus en effectuant une opération logarythmique Laplacienne de Gaussienne LoG et en réalisant une segmentation grossière sur la ROI qui soutient l'opération LoG, pour former des régions grossièrement segmentées sur la ROI, et pour supprimer la région de fluide amniotique de la ROI, l'unité de segmentation (30) étant en outre configurée pour segmenter les régions grossièrement segmentées en sous-régions homogènes obtenues en réunissant des régions voisines grossièrement segmentées sur base d'une distance qui les sépare en effectuant une segmentation fine.

4. Système d'imagerie ultrasonique selon la revendication 3, dans lequel l'unité de classification et de groupage (40) sur base de la texture est configurée pour segmenter chacun des keyframes en blocs non superposés de différentes dimensions et pour calculer différentes probabilités inverses BDIP et des coefficients BVLC de moments de variation de blocs et de corrélation locale dans chaque bloc pour mesurer la texture des sous-régions obtenues par segmentation fine, l'unité de classification et de groupage (40) sur base de la texture étant en outre configurée pour classer les sous-régions en sous-régions à foetus et en sous-régions sans foetus en utilisant un vecteur support de machine SVM, dans lequel BDIP est défini comme étant le ratio de la somme de valeurs qui sont obtenues en soustrayant le nombre de pixels de la valeur maximale de pixels dans le bloc, par rapport au nombre maximal de pixels dans le bloc, et BVLC étant défini comme une variation des coefficients de corrélation locale dans le bloc selon quatre directions à -90 °, 0 °, -45 et 45 °.

5. Procédé pour former une image ultrasonique, comprenant :

   a) fournir des données de volume en 3D d'un foetus et d'une région voisine du foetus, dans lequel les données de volume en 3D sont constituées d'un nombre de trames ;
   b) sélectionner un nombre prédéterminé de trames autour d'une trame centrale parmi les trames considérées comme keyframes ;

c) segmenter chacune des trames en régions de foetus et en régions sans foetus ;

d) classifier les régions à foetus et les régions sans foetus en sous-régions à foetus et en sous-régions sans foetus, sur base de leur texture, et supprimer les sous-régions sans foetus dans les keyframes et rassembler les sous-régions à foetus ;

e) extraire les contours de foetus dans les écrans clés, les sous-régions sans foetus étant supprimés et déterminer une surface 3D du foetus en connectant les contours extraits des keyframes; et

f) former un volume masqué avec la surface 3D et former une image ultrasonique en 3D du foetus en restituant le volume masqué.

6. Procédé selon la revendication 5, dans lequel les régions sans foetus comprennent les régions à fluide amniotique et la région de l'abdomen du corps d'une mère.

7. Procédé selon la revendication 6, dans lequel l'étape c) inclut :

la détermination d'une région présentant un intérêt ROI dans un des keyframes ;

l'application de la ROI à chacun des keyframes ;

la segmentation de la ROI dans chacun des keyframes dans les régions à foetus et les régions sans foetus en effectuant une opération LoG Laplacienne de Gaussienne ;

la réalisation d'une segmentation grossière sur la ROI qui soutient l'opération du LoG, pour former des régions grossièrement segmentées et pour supprimer la région de fluide amniotique de la ROI, et

la segmentation des régions grossièrement segmentées en sous-régions homogènes obtenues en réunissant des régions voisines grossièrement segmentées sur base d'une distance qui les sépare en effectuant une segmentation fine.

8. Procédé selon la revendication 7, dans lequel l'étape d) inclut :

la segmentation de chacun des keyframes en blocs non superposés de différentes dimensions ;

le calcul de différentes probabilités inverses BDIP et de variation de bloc de corrélation locale de moments BVLC en bloc capturé pour mesurer la texture des sous-régions obtenues par segmentation fine, dans lequel BDIP est défini comme étant le ratio de la somme de valeurs qui sont obtenues en soustrayant le nombre de pixels de la valeur maximale de pixels dans le bloc, par rapport au nombre maximal de pixels dans le bloc, et BVLC étant défini comme une variation des coefficients de corrélation locale dans le bloc selon quatre directions à -90 °, 0 °, -45 ° et 45 °; et

la classification des sous-régions en sous-régions à foetus et en régions sans foetus en utilisant une machine à vecteur support SVM.

# FIG. 1

EP 1 793 350 B1

## FIG. 2A

## FIG. 2B

FIG. 3A

FIG. 3B

FIG. 3C

FIG. 3D

FIG. 3E

FIG. 4A

FIG. 4B

FIG. 5

FIG. 6

FIG. 7A

FIG. 7B

FIG. 7C

FIG. 7D

FIG. 8A

FIG. 8B

FIG. 9A

FIG. 9B

FIG. 10A

FIG. 10B

FIG. 11A

FIG. 11B

FIG. 12A

FIG. 12B

# FIG. 13

## FIG. 14A
## (PRIOR ART)

## FIG. 14B

## FIG. 15A
## (PRIOR ART)

## FIG. 15B

FIG. 16A
(PRIOR ART)

FIG. 16B

# FIG. 17A
# (PRIOR ART)

# FIG. 17B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102005116319

- EP 1582150 A1

**Non-patent literature cited in the description**

- Three-Dimensional Ultrasound Imaging. **THOMAS R NELSON ; DOLORES H PRETORIUS.** Ultrasound in Med. & Biol. Elsevier, 1998, vol. 24, 1243-1270

- An Automatic Body ROI Determination for 3D Visualization of a Fetal Ultrasound Volume. **TIEN DUNG NGUYEN et al.** Proceedings KES 2005. Springer, 14 September 2005, 145-153